(19) Europäisches Patentamt
European Patent Office
Office européen des brevets

(11) Veröffentlichungsnummer : **0 569 331 A2**

(12) # EUROPÄISCHE PATENTANMELDUNG

(21) Anmeldenummer : **93810307.4**

(22) Anmeldetag : **27.04.93**

(51) Int. Cl.[5] : **C07C 41/03,** C07C 43/13

(30) Priorität : **06.05.92 CH 1455/92**

(43) Veröffentlichungstag der Anmeldung :
**10.11.93 Patentblatt 93/45**

(84) Benannte Vertragsstaaten :
**CH DE ES FR GB IT LI NL**

(71) Anmelder : **CIBA-GEIGY AG**
**Klybeckstrasse 141**
**CH-4002 Basel (CH)**

(72) Erfinder : **Wolleb, Heinz, Dr.**
**Chesalles**
**CH-1723 Marly (CH)**
Erfinder : **Hafner, Andreas, Dr.**
**Bendenweg 3**
**CH-3177 Laupen (CH)**
Erfinder : **Rolfe, William Martin**
**8 Trinity Close**
**Balsham, Cambridgeshire (GB)**

(54) **Verfahren zur Herstellung von Anlagerungsprodukten.**

(57) Verfahren zur Herstellung von Anlagerungsprodukten durch Umsetzung eines Alkohols mit einer Epoxidverbindung in Gegenwart eines Katalysators, dadurch gekennzeichnet, dass man einen Alkohol der Formel I

$$A\left[-OH\right]_r \qquad (I),$$

worin A einen aliphatischen, cycloaliphatischen oder araliphatischen Rest bedeutet und r für eine Zahl von 1 bis 10 steht, mit einer Mono- oder Diepoxidverbindung im Äquivalenzverhältnis von 1 :20 bis 20 :1, bezogen auf die Hydroxyl- und Epoxidgruppen, in Gegenwart einer Metallkomplexverbindung der Formel II als Katalysator

$$[M^{n+}(L_1)_x(L_2)_y(L_3^{m-})_z][X^{k-}]_{(n-zm)/k} \qquad (II),$$

als Katalysator umsetzt, worin
M ein Metall der Haupt- oder Nebengruppen des Periodensystems,
$L_1$ und $L_2$ schwachgebundene, neutrale, uni- oder multidentale Liganden,
$L_3$ ein starkgebundener, nichtaustauschbarer neutraler oder anionischer, uni- oder multidentaler Ligand,
X ein Anion der folgenden Formeln $BF_4^-$, $PF_6^-$, $AsF_6^-$, $SbF_6^-$, $Clo_4^-$, $JO_4^-$, $NO_3^-$ oder den Sulfonatrest einer perfluorhaltigen Alkansulfonsäure,
n eine Zahl von 1 bis 6,
m null oder eine Zahl von 1 bis 6,
k die Zahl 1 oder 2,
x eine Zahl von 1 bis 1000,
y null oder eine Zahl von 1 bis 1000 und
z null oder eine Zahl von 1 bis 6 bedeuten.

EP 0 569 331 A2

Die vorliegende Erfindung betrifft ein Verfahren zur Herstellung von Anlagerungsprodukten durch Umsetzung von aliphatischen, cycloaliphatischen oder araliphatischen Alkoholen mit Mono- oder Diepoxidverbindungen in Gegenwart bestimmter Metallkomplexe als Katalysatoren.

Im US-Patent 4,543,430 wird ein Verfahren zur Herstellung von Anlagerungsprodukten aus Alkylenoxid oder Epichlorhydrin und Hydroxylgruppen aufweisenden Verbindungen in Gegenwart von Tetraalkylammonium-triflat oder einem bestimmten Metallsalz der Trifluormethansulfonsäure offenbart, die aufgrund ihrer Herstellung zu den Kristallwasser enthaltenden Aquakomplexen zählen.

In der EP-A-0 139 042 werden ebenfalls Kristallwasser enthaltende Aquakomplexe von Metallsalzen bestimmter Sulfonsäuren als Katalysatoren für die Vorverlängerungsreaktion von Epoxidharzen mit Alkoholen eingesetzt.

Für die Herstellung von Anlagerungsprodukten aus Epoxidverbindungen und Alkoholen wird im GB-Patent 2 166 738 die Verwendung von Metallperchloraten als Katalysatoren vorgeschlagen.

Auch in der DE-OS 26 35 564 werden zur Herstellung von Anlagerungsprodukten aus Epoxidverbindungen und Alkoholen Natriumfluorborat und bestimmte Metallperchlorate, die zum Teil Kristallwasser enthaltende Aquakomplexe sind, eingesetzt.

Es wurde nun gefunden, dass die Umsetzung von aliphatischen, cycloaliphatischen oder araliphatischen Alkoholen mit Mono- oder Diepoxidverbindungen unter Verwendung bestimmter Katalysatorsysteme auf der Basis von Metallkomplexen, die vollständig oder teilweise andere Liganden als Wasser enthalten, viel selektiver verläuft und vergleichsweise bei der Glycidyletherherstellung zu Glycidylethern mit niedrigeren Chlor- und höheren Epoxidwerten führt.

Gegenstand der vorliegenden Erfindung ist daher ein Verfahren zur Herstellung von Anlagerungsprodukten durch Umsetzung eines Alkohols mit einer Epoxidverbindung in Gegenwart eines Katalysators, dadurch gekennzeichnet, dass man einen Alkohol der Formel I

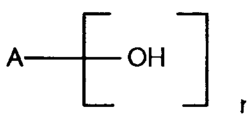

(I),

worin A einen aliphatischen, cycloaliphatischen oder araliphatischen Rest bedeutet und r für eine Zahl von 1 bis 10 steht, mit einer Mono- oder Diepoxidverbindung im Äquivalenzverhältnis von 1:20 bis 20:1, bezogen auf die Hydroxyl- und Epoxidgruppen, in Gegenwart einer Metallkomplexverbindung der Formel II als Katalysator

$$[M^{n+}(L_1)_x(L_2)_y(L_3^{m-})_z][X^{k-}]_{(n-zm)/k} \qquad (II),$$

umsetzt, worin

M ein Metall der Haupt- oder Nebengruppen des Periodensystems,

$L_1$ und $L_2$ schwachgebundene, neutrale, uni- oder multidentale Liganden,

$L_3$ ein starkgebundener, nichtaustauschbarer neutraler oder anionischer, uni- oder multidentaler Ligand,

X ein Anion der folgenden Formeln $BF_4^-$, $PF_6^-$, $AsF_6^-$, $SbF_6^-$, $ClO_4^-$, $JO_4^-$, $NO_3^-$ oder den Sulfonatrest einer perfluorhaltigen Alkansulfonsäure,

n eine Zahl von 1 bis 6,

m null oder eine Zahl von 1 bis 6,

k die Zahl 1 oder 2,

x eine Zahl von 1 bis 1000,

y null oder eine Zahl von 1 bis 1000 und

z null oder eine Zahl von 1 bis 6 bedeuten.

In Formel I kann A als aliphatischer Rest geradkettig oder verzweigt, gesättigt oder ungesättigt sein und gegebenenfalls durch ein oder mehrere Sauerstoff- oder Schwefelatome in der Kette unterbrochen sein oder eine oder mehrere Ketogruppen enthalten.

Der cycloaliphatische Rest A kann gesättigt oder ungesättigt sein und ein oder mehrere Ringsysteme aufweisen und gegebenenfalls eine Ketogruppe enthalten, wobei die Ringe durch Alkylgruppen substituiert und/oder überbrückt sein können.

Der aromatische Rest im araliphatischen Rest A kann einen oder mehrere, gegebenenfalls ankondensierte Ringe aufweisen, wie z.B. Phenyl, Naphthyl, 4,4'-Diphenyl-, 4,4'-Diphenylmethan-, 4,4'-Diphenyl-(dimethyl)methan-, 4,4'-Diphenyloxyd-, -keton- oder -sulfon-Reste, welche ihrerseits durch $C_1$-$C_6$Alkyl, $C_1$-$C_6$Alkoxy oder Halogen substituiert sein können. Araliphatische Reste sind insbesondere Aralkylreste, in denen das Alkyl unverzweigt oder verzweigt sein kann und vorzugsweise 1 bis 3 C-Atome aufweisen.

$C_1$-$C_6$Alkyl und $C_1$-$C_6$Alkoxy können geradkettig oder verzweigt sein und bedeuten z.B. Methyl, Ethyl, Propyl,

Isopropyl, Butyl, sek.-Butyl, tert.-Butyl, Pentyl und Hexyl bzw. Methoxy, Ethoxy, Propoxy, Isopropoxy, Butoxy, tert.-Butoxy, Pentoxy und Hexyloxy. Halogen kann Chlor, Brom oder Iod sein, bevorzugt aber ist es Brom und insbesondere Chlor.

Der Rest A kann gegebenenfalls mit funktionellen Gruppen substituiert sein, vorausgesetzt, dass sie den eingesetzten Katalysator nicht inhibieren und keine Nebenreaktionen mit den Epihalohydrinen eingehen.

Der Rest A als Alkylrest, wenn r=1, bedeutet z.B. $C_1$-$C_{30}$Alkyl, bevorzugt $C_3$-$C_{20}$Alkyl. Beispiele für solche Reste sind Methyl, Ethyl, n-Propyl, Isopropyl, n-Butyl, sek.-Butyl, tert.-Butyl, Pentyl, Hexyl, Heptyl, Octyl, 2-Ethylhexyl, Nonyl, Decyl, Dodecyl, Tetradecyl, Hexadecyl, Octadecyl, Eicosyl, Docosyl, Tetracosyl und Pentacosyl.

Cycloalkylreste sind beispielsweise Cyclopentyl, Cyclohexyl, Cycloheptyl, Cyclooctyl und Cyclononyl, insbesondere aber Cyclohexyl.

Beispiele für mehrfunktionelle Alkohole der Formel I sind solche mit aliphatischen, cycloaliphatischen oder araliphatischen Strukturelementen, wie zum Beispiel Ethylenglykol, Propan-1,2-diol, Propan-1,3-diol, Butan-1,4-diol, Butan-1,2-diol, Butan-2,3-diol, Diethylenglykol, Triethylenglykol, Pentan-1,5-diol, Hexan-1,6-diol, Hexan-2,4,6-triol, 2,2-Dimethyl-1,3-propandiol, 2-Ethyl-2-butylpropan-1,2-diol, 1,12-Dihydroxyoctadecan, Glycerin, Trimethylolpropan, Erythrit, Pentaerythrit, Sorbit, Mannit, Inosit, 1,1,1 -Trimethylolpropan, 1 ,4-Dimethylolbenzol, 4,4'-Dimethyloldiphenyl, Dimethylolxylole, Dimethylolnaphthaline, Polyetheralkohole, wie Diglycerin, ferner Bis-(2,3-dihydroxypropylether), Triglycerin, Dipentaerythritol, Dimethylolanisole, beta-Hydroxyethylether von Polyalkoholen oder Phenolen, wie Diethylenglykol, Polyethylenglykol oder Hydrochinon-bis-(beta-hydroxyethylether), Bis-(beta-hydroxyethylether) von Bisphenolen, wie von 4,4'-Dihydroxydiphenyl-dimethylmethan, ferner beta-Hydroxyethylether von Glycerin, Pentaerythrit, Sorbit oder Mannit, Kondensate von Alkylenoxyden, wie Ethylen-, Propylen-, Butylen- oder Isobutylenoxyd, mit den oben genannten Polyalkoholen, ferner Hydroxyester, wie z.B. Monoglyceride, wie Monostearin, ferner Ethylenglykoldilactat, Monoester von Pentaerythrit, wie das Monoacetat, ferner halogenierte Alkohole, wie Glycerinmonochlorhydrin, 1,4-Dichlor-2,3-dihydroxybutan,, Pentaerythritmonochloride oder Dibromneopentylglykol, Bis-(4-hydroxycyclohexyl)-methan, 2,2-Bis-(4-hydroxycyclohexyl)-propan (Perhydrobisphenol A), Cyclohexan-1,1-dimethylol, 2,2,6,6-Tetramethylol-cyclohexanol, 2,2,5,5-Tetramethylol-cyclopentanol, 4-Methyl-2,2,6,6-tetramethylolcyclohexanol, 2,2,6,6-Tetramethylol-cyclohexanon-4, 1,2-, 1,3- und 1,4-Dihydroxycyclohexan, 1,3-Dihydroxycyclopentan, 4,4'-Dihydroxydicyclohexyl, ferner Mercaptoalkohole, wie 2-Mercaptoethanol, alpha-Monothioglycerin, 2,2',3,3'-Tetrahydroxydipropylsulfid oder 2,2'-Dihydroxydiethylsulfid.

Vorzugsweise stellt A in der Formel I einen aliphatischen oder cycloaliphatischen Rest, insbesondere einen aliphatischen Rest, dar.

In der Formel I ist r bevorzugt eine Zahl von 1 bis 6.

Erfindungsgemäss besonders bevorzugte Verbindungen der Formel I sind primäre oder sekundäre polyfunktionelle Alkohole, bei welchen r eine Zahl von 2 bis 6 darstellt, und ganz besonders bevorzugt ist A ein polyfunktioneller Rest mit bis zu 30 C-Atomen, wobei r eine ganze Zahl von 2 bis 6 ist.

Als Monoepoxide können beim erfindungsgemässen Verfahren alle gebräuchlichen Monoepoxide eingesetzt werden, doch verwendet man als solche vorzugsweise ein Epihalohydrin, wie beispielsweise Epichlorhydrin, β-Methylepichlorhydrin oder Epibromhydrin und insbesondere Epichlorhydrin, oder ein Alkylenoxid, wie beispielsweise Ethylenoxid, 1,2-Propylenoxid oder 1,2-Butylenoxid.

Als Diepoxidverbindungen können beim erfindungsgemässen Verfahren ebenfalls die gebräuchlichen Diepoxidverbindungen eingesetzt werden. Beispielsweise sind dafür die Diglycidylether der zuvor genannte Diole oder die Diglycidylether von Diphenolen, wie beispielsweise Resorcin, Hydrochinon oder 4,4'-Dihydroxybiphenyl, vorzugsweise von Bisphenolen, wie beispielsweise Bis-(4-hydroxyphenyl)-methan, Bis-(4-hydroxyphenyl)-säure, von cycloaliphatischen Dicarbonsäuren, wie beispielsweise Tetrahydrophthalsäure, 4-Methyltetrahydrophthalsäure oder Hexahydrophthalsäure oder von aromatischen Dicarbonsäuren, wie beispielsweise Phthalsäure, Isophthalsäure oder Terephthalsäure, geeignet. Ferner können beim erfindungsgemässen Verfahren auch cycloaliphatische Epoxidharze, wie beispielsweise Bis-(2,3-epoxycyclopentyl)-ether, 2,3-Epoxycyclopentylglycidylether, 1,2-Bis-(2,3-epoxycyclopentyloxy)-ethan oder 3,4-Epoxycyclohexylmethyl-3,4'-epoxycyclohexancarboxylat.

Vorzugsweise setzt man beim erfindungsgemässen Verfahren ein Monoepoxid, wie ein Epihalogenhydrin oder ein Alkylenoxid, insbesondere ein Epihalogenhydrin, ein.

Die Anlagerung von Epihalogenhydrinen an die Alkohole der Formel I führt zu Halogenhydrinethern, die zweckmässig nicht isoliert, sondern mit einer Base direkt zu den Glycidylethern der Formel III

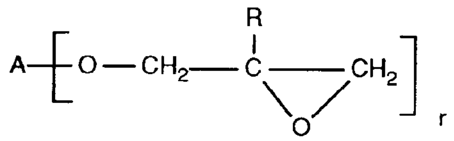

(III),

dehydrohalogniert werden, worin A und r die gleiche Bedeutung wie in Formel I haben, und R für ein Wasserstoffatom oder Methyl steht.

Gegenstand vorliegender Erfindung ist somit auch ein Verfahren zur Herstellung von Verbindungen der Formel III, dadurch gekennzeichnet, dass man die durch Anlagerung von Epihalogenhydrin an die Alkohole der Formel I in Zwischenstufe erhaltenen Halogenhydrinether anschliessend mittels einer Base zu den Glycidylethern der Formel III dehydrohalogeniert.

Bei der Anlagerung von Epihalogenhydrinen an die Alkohole der Formel I setzt man als Epihalogenhydrin vorzugsweise Epichlorhydrin ein.

Das Epihalogenhydrin wird dabei zweckmässig in solchen Mengen eingesetzt, dass zwischen 0,7 und 2,0 Mol Epihalogenhydrin pro Äquivalent Hydroxylgruppe der Verbindung der Formel I vorliegen. Bevorzugt werden aber stöchiometrischen Mengen, das heisst 0,9 bis 1,1 Mol Epihalogenhydrin pro Äquivalent Hydroxylgruppe eingesetzt.

Die beim erfindungsgemässen Verfahren verwendeten Metallkatalysatoren der Formel III sind bekannt, beispielsweise aus Gmelin, Band 39, Teil C5, Seite 39 ff oder Inorganic Chemistry, 21, Seite 2867 ff (1982) und können beispielsweise hergestellt werden, indem man ein Metalloxid oder ein Metallsalz, wie beispielsweise ein Metallhalogenid oder -carbonat, mit der das Anion X bildenden Säure in Gegenwart einer den Liganden $L_1$ und gegebenenfalls die Liganden $L_2$ und/oder $L_3$ bildenden Verbindung umsetzt.

Als Verbindungen, welche den Liganden $L_1$ bilden, eignen sich beispielsweise Nitrile, Isonitrile, Thioether, Phosphine, Ether, Aldehyde, Ketone oder $sp^3$-stickstoffhaltige Verbindungen und als Verbindungen, welche den Liganden $L_2$ bilden, eignen sich beispielsweise Wasser, Nitrile, Isonitrile, Thioether, Phosphine, Ether, Aldehyde, Ketone oder $sp^3$-stickstoffhaltige Verbindungen.

Als Verbindungen, die den Liganden $L_3$ bilden, eignen sich beispielsweise Phosphine, Phosphite, Phosponite, Halogenide, Cyanide, Alkoholate, Thiolate, Carboxylate, Acetylacetonat, ein unsubstituiertes oder durch ein oder mehrere $C_1$-$C_4$-Alkyle substituiertes Cyclopentadienyl oder $sp^2$-stickstoffhaltige Verbindungen.

Als Nitrile für die Liganden $L_1$ und $L_2$ eignen sich beispielsweise aliphatische, araliphatische oder aromatische Nitrile, wie zum Beispiel Acetonitril, Propionitril, n-Butyronitril, Isobutyronitril, Valeronitril, Tetradecanonitril, Malononitril, Succinonitril, Glutaronitril, Acrylonitril, Methacrylonitril, Allylcyanid, Phenylacetonitril, Benzoylcyanid, Benzonitril, 2-Chlor-, 4-Chlor- oder 2,6-Dichlorbenzonitril, 2-Chlor-4-nitro-benzonitril, 4-Hydroxybenzonitril oder 1-Cyanonaphthalen.

Als Isonitrile (Isocyanide) für die Liganden $L_1$ und $L_2$ eignen sich ebenfalls aliphatische, araliphatische oder aromatische Isonitrile, wie zum Beispiel Methylisocyanid, n-Butylisocyanid, Phenylisocyanid oder 3-Chlorphenylisocyanid.

Als Thioether (organische Sulfide) für die Liganden $L_1$ und $L_2$ eignen sich beispielsweise aliphatische, araliphatische oder aromatische Thioether, wie beispielsweise Diethylsulfid, Dripropylsulfid, Ethylphenylsulfid, Diphenylsulfid, Benzylphenylsulfid, Dibenzylsulfid oder 4-(Ethylthio)-phenol.

Als Phosphine für die Liganden $L_1$ und $L_2$ seien beispielsweise aliphatische, araliphatische oder aromatische Phosphine genannt. Diese können primäre, sekundäre oder tertiäre Phosphine sein, je nachdem, ob 1, 2 oder 3 H-Atome des Phosphorwasserstoffs, $PH_3$, durch organische Gruppen substituiert sind. Beispiele für Phosphine sind Trimethylphosphin, Triethylphosphin, Tribenzylphosphin oder Triphenylphosphin.

Als Ether für die Liganden L1 und $L_2$ seien beispielsweise aliphatische, araliphatische, cycloaliphatische oder aromatische Ether genannt, wie beispielsweise Dimethylether, Diethylether, Di-n-propylether, Diisopropylether, Di-n-butylether, Bis-(chlormethyl)-ether, Bis-(chlorethyl)-ether, Methyl-n-propylether, Methyl-tert.-butylether, Vinylether, wie Methyl- oder Ethylvinylether, Allylether, wie Allylphenylether; Benzylphenylether, Furan, Tetrahydrofuran, Dioxan, Kronenether, wie 18-Krone-6, 15-Krone-5, oder 12-Krone-4; Phenylmethylether, Phenylethylether oder Diphenylether.

Als Aldehyde für die Liganden $L_1$ und $L_2$ seien beispielsweise aliphatische, araliphatische oder aromatische Ether genannt, wie beispielsweise Formaldehyd (Methanal), Acetaldehyd (Ethanal), Butyraldehyd (Butanal), 2-Methylbutanal, 2,2-Dimethylpropanal, 2-Ethylhexanal; 2,2-Dimethylpentanal, 2-Hydroxyethanal, 2-Phenylacetaldehyd, 2-Phenylpropanal, 3-Phenyl-2-propanal (Cinnamaldehyd), Benzaldehyd oder Anisaldehyd.

Stellen die Liganden $L_1$ und $L_2$ ein Keton dar, so handelt es sich dabei um eine aliphatische, araliphatische, cycloaliphatische oder aromatische Verbindung mit vorzugsweise einer oder zwei Ketogruppen im Molekül, wie beispielsweise Aceton, Methylethylketon, Ethylpropylketon, Diisopropylketon, Acetophenon,

Propiophenon, Chalcon, Desoxybenzoin, Hexan-2,4-dion, 1,4-Benzochinon, Cyclopentanon, Cyclohexanon, 1,4-Naphthochinon, Anthrachinon oder Benzophenon.

Es sind für $L_1$ und $L_2$ auch Liganden möglich, die mehrere unterschiedliche Ligandenstellen in einem Molekül besitzen, wie beispielsweise Methoxyacetaldehyd, 2-Methoxyacetophenon oder 2-Methoxybenzaldehyd.

Stellen die Liganden $L_1$ und $L_2$ eine $sp^3$-stickstoffhaltige Verbindung dar, so handelt es sich hierbei um tertiäre, keine Doppelbindung aufweisende Stickstoffverbindungen, wie beispielsweise Trimethylamin, Triethylamin, Tributylamin, Triisobutylamin, Tripentylamoin oder Triphenylamin.

Die für die Liganden $L_1$ und $L_2$ genannten Verbindungen sind bekannt und grösstenteils im Handel erhältlich.

Als Verbindungen, welche den Ligand $L_3$ bilden, eignen sich beispielsweise Phosphine, Phosphite, Phosphonite, Halogenide, Cyanide, Alkoholate, Thiolate, Carboxylate, Acetylacetonate, unsubstituiertes oder durch ein oder mehrere $C_1$-$C_4$-Alkyle oder Tri-($C_1$-$C_4$-alkyl)-silyl substituierte Cyclopentadienyle oder $sp^2$-stickstoffhaltige Verbindungen.

Steht der Ligand $L_3$ für ein Phosphin, so sind beispielsweise die unter den Linganden $L_1$ und $L_2$ genannten aliphatischen, araliphatischen oder aromatischen Phosphine geeignet.

Bedeutet der Ligand $L_3$ ein Phosphit, so handelt es sich hierbei um Anionen der phosphorigen Säure, $P(OH)_3$ oder um dieEster der phosphorigen Säure, wie beispielsweise Dimethyl-, Diethyl-, Trimethyl- oder Triethylphosphit.

Bedeutet der Ligand $L_3$ ein Phosphonit, so handelt es sich hierbei um Anionen und Ester der phosphonigen Säure, $HP(OH)_2$, insbesondere der organisch substituierten phosphonigen Säure, wie methyl-, octyl- oder phenylphosphonige Säure.

Steht der Ligand $L_3$ für ein Halogenid, so handelt es sich um $F^-$,$Cl^-$,$Br^-$ oder $J^-$.

Bedeutet der Ligand $L_3$ ein Cyanid, so handelt es sich um Cyanid-Anion.

Steht der Ligand $L_3$ für ein Thiolat (Mercaptid), so handelt es sich um ein Anion der Thiole, R-SH, worin R für eine Alkyl- oder Arylgruppe steht.

Bedeutet der Ligand $L_3$ ein Carboxylat, so handelt es sich hierbei um ein Anion von gesättigten oder ungesättigten aliphatischen Monocarbonsäuren, wie beispielsweise Essigsäure, Propionsäure, Buttersäure, Capronsäure, Laurinsäure, Palmitinsäure, Acrylsäure, Methacrylsäure, Propiolsäure, Crotonsäure, Sorbinsäure oder Ölsäure, von cycloaliphatischen Monocarbonsäuren, wie Cycylohexancarbonsäure, von aromatischen Carbonsäuren, wie Benzoesäure, Naphthoesäuren oder Tolylsäuren, oder von araliphatischen Monocarbonsäuren,wie beispielsweise Hydrotropasäure, Atropasäure oder Zimtsäuren.

Steht der Ligand $L_3$ für eine $sp^2$-stickstoffhaltige Verbindung, so handelt es sich hierbei um eine die -N=C-Bindung enthaltende Verbindung, wie beispielsweise Azomethine, wie Benzalanilin oder Benzalimin.

Als Metallkatalysatoren verwendet man beim erfindungsgemässen Verfahren vorzugsweise solche, worin in der Formel III

A ein Metall der II., III. oder IV. Hauptgruppe des Periodensystems, ein Metall der Übergangsgruppen, ein Lanthanid oder Actinid,

$L_1$ ein Nitril, Isonitril, Thioether, Phosphin, Ether, Aldehyd, Keton oder eine $sp^3$-stickstoffhaltige Verbindung,

$L_2$ ein Wassermolekül, Nitril, Isonitril, Thioether, Phosphin, Ether, Aldehyd, Ketone oder eine $sp^3$-stickstoffhaltige Verbindung,

$L_3$ ein Phosphin, Phosphit, Phosponit, Halogenid, Cyanid, Alkoholat, Thiolat, Carboxylat, Acetylacetonat, ein unsubstituiertes oder durch ein oder mehrere $C_1$-$C_4$-Alkyle oder Tri-($C_1$-$C_4$-alkyl)-silyle substituiertes Cyclopentadienyl oder eine $sp^2$-stickstoffhaltige Verbindung,

X ein Anion der folgenden Formeln $BF_4^-$, $PF_6^-$, $AsF_6^-$, $SbF_6^-$, $ClO_4^-$, $JO_4^-$, $NO_3^-$ oder den Sulfonatrest einer perfluorhaltigen Alkansulfonsäure,

n eine Zahl von 1 bis 3,

m null oder eine Zahl von 1-3,

k die Zahl 1 oder 2,

x eine Zahl von 1 bis 10,

y null oder eine Zahl von 1 bis 10 und

z null oder eine Zahl von 1 bis 3 bedeuten.

X als Sulfonatrest, also Anion einer perfluorhaltigen Alkansulfonsäure kann beispielsweise für eines der folgenden Anionen stehen: $CF_3SO_3^-$, $C_8F_{17}SO_3^-$, $CF_3C_6F_{10}SO_3^-$, $C_3F_7SO_3^-$, $C_2F_5SO_3^-$,$C_2HF_4SO_3^-$, $C_3F_7CHFCF_2SO_3^-$, $(CF_3)_2CHCF_2SO_3^-$, $C_4F_7SO_3^-$, $(CF_3)_2CF(CF_2)_4SO_3^-$, $C_4F_9CFHCF_2SO_3^-$, $C_3F_7CH(CF_3)CF_2SO_3^-$, $C_{11}F_{23}SO_3^-$, $C_5H_{11}CFHCF_2SO_3^-$ oder $C_7F_{15}CFHCF_2SO_3^-$. Vorzugsweise steht X für $CF_3SO_3^-$.

Insbesondere verwendet man beim erfindungsgemässen Verfahren Metallkatalysatoren der Formel II, worin

$L_2$ ein Wassermolekül, Nitril, Isonitril, Thioether oder Ether,

$L_3$ ein Phosphin, Phosphit, Halogenid oder Carboxylat,
X ein Anion der folgenden Formeln $BF_4^-$, $PF_6^-$, $ClO_4^-$ oder den Trifluormethansulfonatrest bedeuten.

Für das erfindungsgemässe Verfahren stellen $La(CH_3CN)_x(H_2O)_y(CF_3SO_3)_3$ und $La(CH_3CN)_x(H_2O)_y(ClO_4)_3$ besonders geeignete Katalysatoren der Formel II dar.

Die Metallkatalysatoren der Formel II können, ohne die Umsetzung ungünstig zu beeinflussen, in einem weiten Konzentrationsbereich verwendet werden, jedoch ist eine Konzentration von 0,001 bis 0,5 Mol des Katalysators auf 1 Mol Alkohol der Formel I vorteilhaft.

Der Katalysator kann entweder separat oder in situ hergestellt werden.

Die Umsetzung der Verbindungen der Formel I mit den Mono- und Diepoxiden erfolgt zweckmässig ohne Lösungsmittel, sie kann aber auch in Gegenwart eines inerten organischen Lösungsmittels durchgeführt werden. Geeignete Lösungsmittel sind z.B. halogenierte Kohlenwasserstoffe, wie Methylenchlorid, Chloroform, 1,2-Dichlorethan, 1,1,1 -Trichlorethan, 1,2,2-Trichlorethylen, 1,4-Dichlorpropan und Chlorbenzol, ferner Toluol, Xylol, Hexan, Cyclohexan, Heptan, Octan, ferner Ester, wie Ethylacetat und Butylacetat, Ether, wie Diethyl- und Diisopropylether, Dioxan und Tetrahydrofuran. Auch Lösungsmittelgemische obiger Lösungsmittel in beliebigen Mengenverhältnissen können verwendet werden.
Die Menge des Lösungsmittels bzw. des Lösungsmittelgemisches kann beliebig variieren. Sie wird zweckmässig so gewählt, dass die Reaktandenkonzentrationen z.B. zwischen 10 und 70 Gew.% liegen.

Man führt die Umsetzung des Alkohols mit dem Mono- oder Diepoxid zweckmässig zwischen 0 °C und der Rückflusstemperatur des Reaktionsgemisches, vorzugsweise zwischen 60 und 130 °C, durch. Die Gesamtreaktionszeiten variieren je nach Alkohol und Katalysator zwischen 30 Minuten und 48 Stunden. Gegebenenfalls kann unter erhöhtem oder reduziertem Druck gearbeitet werden.

Bei der Durchführung des erfindungsgemässen Verfahrens ist die Reihenfolge der Zugabe der Komponenten nicht wesentlich. So können zum Beispiel der Alkohol und der Bei der Durchführung des erfindungsgemässen Verfahrens ist die Reihenfolge der Zugabe der Komponenten nicht wesentlich. So können zum Beispiel der Alkohol und der Katalysator vorgelegt und anschliessend die Epoxyidverbindung zudosiert werden, oder es werden alle Reaktionskomponenten vorgelegt und so zur Reaktion gebracht. Man lässt so lange reagieren, bis die analytische Untersuchung des Reaktionsgemisches den gewünschten Umsatz anzeigt.

Mögliche Verfahrensvarianten sind beispielsweise bei der bevorzugtem Ausführungsform des erfindungsgemässen Verfahrens, nämlich der Umsetzung des Alkohols der Formel I mit Epichlorhydrin: Der Alkohol wird zusammen mit dem Katalysator vorgelegt und auf die benötigte Reaktionstemperatur aufgeheizt. Das Epihalohydrin wird dann innerhalb geeigneter Zeitperioden zudosiert, so dass die Temperatur innerhalb eines geeigneten Bereiches bleibt. Dann lässt man solange weiterreagieren, bis die analytische Untersuchung des Reaktionsgemisches (z.B. mittels Gaschromatographie, Epoxidtitration oder HPLC) den gewünschten Umsatz zum entsprechenden Halohydrinether anzeigt. Alle Komponenten, d.h. der Alkohol, das Epihalohydrin und der Katalysator, werden vorgelegt, und auf die benötigte Reaktionstemperatur aufgeheizt und bis zum gewünschten Umsatz an Epihalohydrinether weiterreagiert.
Ein eventuell vorhandener Ueberschuss an Epihalohydrin kann vor dem Dehydrohalogenieren destillativ aus dem Reaktionsgemisch entfernt werden.

Die Dehydrohalogenierung des Halohydrinethers zum Glycidylether kann nach den bekannten, üblichen Methoden mit einem Alkalimetallhydroxid als Base unter Abspaltung von Alkalimetallhalogenid erfolgen. Die theoretisch erforderliche stöchiometrische Menge kann dabei gegebenenfalls auch über- oder unterschritten werden, um z.B. die optimalen niedrigen Chlorwerte des Endprodukts zu erreichen. Generell sind Mengen von 0,80 bis 1,30 Mol Alkalimetallhydroxid pro Hydroxylgruppe und Temperaturen zwischen 30 und 60°C bevorzugt. Der Einsatz eines inerten organischen Lösungsmittels ist z.B. angezeigt, wenn die Viskosität der Reaktionsmischung zu hoch ist.
In manchen Fällen kann die Glycidylisierung vorteilhafterweise unter azeotroper Entfernung des Wassers in Gegenwart von Alkalimetallhydroxid durchgeführt werden. Das Alkalimetallhydroxid wird während der azeotropen Entwässerung vorzugsweise unter vermindertem Druck zugesetzt. Wasser, das als Lösungsmittel für das Alkalimetallhydroxid dient, und solches, das bei der Reaktion gebildet wird, wird auf diese Weise laufend aus dem Reaktionsgemisch entfernt.

Das bei der Reaktion gebildete Salz, wie z.B. Kochsalz, kann entweder ausgewaschen, abfiltriert oder abzentrifugiert werden. Ueberschüssiges Epihalohydrin wird gegebenenfalls im Vakuum abdestilliert.

Die erfindungsgemäss als Ausgangsprodukte eingesetzten Verbindungen stellen bekannte Produkte dar und sind zum Teil auch im Handel erhältlich.

Das vorliegende verbesserte Verfahren weist gegenüber vorbekannten Verfahren mehrere Vorteile auf, wie eine bessere Selektivität, was zu niedrigeren Chlor- und höheren Epoxidwerten führt.

Ausserdem kann die erste Reaktionsstufe oft in der Schmelze ohne Lösungsmittel durchgeführt werden.

Die erfindungsgemäss hergestellten Verbindungen werden häufig in Epoxidharz-Formulierungen zur Modifikation bestimmter Eigenschaften eingesetzt, beispielsweise als Reaktivverdünner, Flexibilisatoren oder Adhäsionsverbesserer. Diese Formulierungen enthalten gegebenenfalls zusätzliche andere Epoxidharze, wie zum Beispiel Bisphenol A-Epoxidharze oder Epoxidnovolake, und übliche Härtungsmittel, wie zum Beispiel Amine, Carbonsäureanhydride, Phenole oder katalytische Härter. Die Formulierungen finden Anwendung in verschiedensten Applikationsgebieten, beispielsweise als Lackharze, Tauchharze, Imprägnierharze, Klebstoffe, Dichtungsmittel, Umhüllungsmassen und Isoliermaterialen.
Die erfindungsgemäss erhaltenen Glycidylether weisen aufgrund ihrer aliphatischen Struktur ausgezeichnete Aussenbewitterungsbeständigkeit auf.

Die folgenden Beispiele beschreiben die Erfindung näher. Wenn nichts anderes vermerkt ist, bedeuten Teile Gewichtsteile.

In den Beispielen werden folgende Metallkomplexe eingesetzt, die wie folgt hergestellt werden können.

<u>Katalysator A</u>: $La(CH_3CN)_x(H_2O)_y(CF_3SO_3)_3$

In einem Dreihalsrundkolben, versehen mit Magnetrührer, $N_2$-Überleitung und Septum, werden 3,0 g (5 mMol) $La_2(CO_3)_3(H_2O)_y$ in 50 ml über ein Molekularsieb getrocknetem Acetonitril vorgelegt und anschliessend 4 ml (46 mMol) Trifluormethansulfonsäure zugegeben. Die Suspension wird 24 Stunden (h) bei Raumtemperatur (RT) gerührt, wobei sich eine weisse Paste bildet. Das Reaktionsgemisch wird durch eine Glasfritte filtriert und 4 mal mit 50 ml frisch über Na destilliertem Diethylether gewaschen. Der Rückstand wird 24 h bei RT/$10^{-2}$ T getrocknet. Man erhält 5 g eines weissen Feststoffes, welcher folgende Analysendaten aufweist:

La-Gehalt = 24,9%
C-Gehalt = 9,34%
N-Gehalt = 1,91%.

<u>Katalysator B</u>: $La(H_2O)_y(CF_3SO_3)_3$ mit Benzylphenylsulfid als Ligand $L_1$

In einem Rundkolben, versehen mit einem Magnetrührer, werden 10 ml Wasser vorgelegt und 3,45 g (23 mMol) Trifluormethansulfonsäure zugegeben. Anschliessend gibt man portionenweise 3,0 g (5 mMol) $La_2(CO_3)_3(H_2O)_y$ zu und rührt, bis die Lösung neutral ist. Danach filtriert man die Lösung, und das Filtrat wird am Rotationsverdampfer bei 100°C eingedampft. Man erhält 5,6 g $La(H_2O)_y(CF_3SO_3)_3$ als weissen Feststoff, welcher einen La-Gehalt von 24,5% aufweist. 1,38 g des Feststoffes werden in 200 ml Toluol suspendiert und unter Hinzufügen von 0,6 g (3 mMol) Benzylphenylsulfid bei 100°C erhält man den Katalysator B, der direkt in Beispiel 2 eingesetzt wird.

<u>Katalysator C</u>: $La(CH_3CN)_x(H_2O)_y(ClO_4)_3$

In einem Rundkolben, versehen mit Magnetrührer, werden 8 ml Wasser und 788 mg (5,5 mMol) 70%ige, wässrige Perchlorsäure vorgelegt. Anschliessend wird 1 g $La_2(CO_3)_3(H_2O)_y$ zugegeben und gerührt, bis die Suspension neutral reagiert. Danach wird filtriert und das Filtrat in einen 750 ml Sulfierkolben, versehen mit Ankerrührer, Thermometer, Dosimat und Destillationsaufsatz, transferiert. Die wässrige Phase wird mit 300 ml Toluol überschichtet und das Gemisch bei einer Ölbadtempeartur von 140°C erwärmt. Sobald die Kopftemperatur des Destillats 105°C erreicht hat, werden 100 ml über ein Molekularsieb getrocknetes Acetonitril zugegeben und das überschüssige Acetonitril abdestilliert. Sobald die Kopftemperatur wiederum 105°C erreicht hat, wird der Vorgang mit 50 ml Acetonitril wiederholt. Diese Katalysatorlösung wird in Beispiel 3 direkt eingesetzt.

<u>Katalyastor D</u>: $La(H_2O)_y(ClO_4)_3$ mit Benzylphenylsulfid als Ligand $L_1$

In einem Rundkolben, versehen mit Magnetrührer, werden 8 ml Wasser und 788 mg (5,5 mMol) 70%ige, wässrige Perchlorsäure vorgelegt. Anschliessend wird 1 g $La_2(CO_3)_3(H_2O)_x$ zugegeben und gerührt, bis die Suspension neutral reagiert. Danach wird filtiert und das Filtrat in einen 750 ml Sulfierkolben, versehen mit Ankerrührer, Thermometer, Dosimat und Destillationsaufsatz, transferiert. Die wässrige Phase wird mit 300 ml Toluol überschichtet und das Gemisch bei einer Ölbadtempeartur von 140°C erwärmt. Sobald die Kopftemperatur des Destillats 105°C erreicht hat, wird der Destillationsaufsatz durch einen Rückflusskühler ersetzt, und es werden 551 mg (2,75 mMol) Benzylphenylsulfid zugegeben. Diese Katalysatorlösung wird in Beispiel 4 direkt eingesetzt.

Katalysator E: $Cu(CH_3CN)_x(H_2O)_yCF_3SO_3$

In einem Dreihalsrundkolben, versehen mit Magnetrührer, $N_2$-Überleitung und Septum, werden 2,15 g (15 mMol) $Cu_2O$ in 50 ml über ein Molekularsieb getrocknetem Acetonitril vorgelegt und anschliessend 5,06 ml (34 mMol) Trifluormethansulfonsäure (Triflat) zugegeben. Die rote Suspension löst sich sofort auf, und es entsteht eine klare, leicht gelbe Lösung, welche zu 150 ml frisch über Na destilliertem Diethylether getropft wird. Es bildet sich ein weisser Niederschlag, welcher abfiltriert und 4 mal mit 50 ml Diethylether gewaschen wird. Das Produkt wird 2 h bei RT/0,013 mbar getrocknet. Man erhält 8,41 g eines weissen Pulvers mit einem Cu-Gehalt von 16,8%.

Katalysator F: $Zn(H_2O)_y(CF_3SO_3)_2$ mit Benzylphenylsulfid als Ligand $L_1$

In einem Rundkolben, versehen mit Magnetrührer, werden 30 ml $H_2O$ und 4,20 g (28 mMol) Trifluormethansulfonsäure vorgelegt und portionenweise 1,22 g (15 Mol) ZnO zugegeben. Sobald die Lösung neutral reagiert, wird das Reaktionsgemisch filtriert und das Filtrat am Rotationsverdampfer bei 100°C vom Lösungsmittel befreit. Man erhält 5,1 g eines weissen Pulvers, welches einen Zn-Gehalt von 17,8% aufweist. 0,94 g des weissen Pulvers werden in 90,12 g (1,0 Mol) Butan-1,4-diol gelöst, dann werden 0,6 g (3 mMol) Benzylphenylsulfid zugegeben. Dieser Katalysator wird direkt in Beispiel 6 eingesetzt.

Katalysator G: $Zn(CH_3CN)_x(H_2O)_y(BF_4)_2$

In einem Dreihalsrundkolben, versehen mit Magnetrührer, $N_2$-Überleitung und Septum, werden 1,63 g (20 mMol) ZnO in 50 ml über ein Molekularsieb getrocknetem Acetonitril vorgelegt, und anschliessend werden 7,9 ml (55 mMol) $HBF_4$.Diethylether (54%) zugegeben. Die Suspension löst sich sofort auf, und es entsteht eine klare, leicht gelbe Lösung, welche zu 300 ml frisch über Natrium destilliertem Diethylether getropft wird. Es bildet sich ein weisser Niederschlag, welcher abfiltriert wird und 4 mal mit 50 ml Diethylether gewaschen wird. Das Produkt wird 24 h bei RT/0,013 bar getrocknet. Man erhält 5,6 g eines weissen Pulvers, welches folgende Analysendaten aufweist:

| | |
|---|---|
| Zn-Gehalt = | 17,30% |
| C-Gehalt = | 16,25% |
| N-Gehalt = | 9,18%. |

Katalysator H: $Fe(CH_3CN)_x(H_2O)_y(CF_3SO_3)_2$

Zu einer Lösung von 15,5 g (0,0356 Mol) Bariumtriflat in 50 ml Wasser werden 9,9 g (0,0356 Mol) $FeSO_4 \cdot 7H_2O$ in 50 ml Wasser zugegeben. Der Niederschlag, bestehend aus $BaSO_4$, wird abfiltriert und das Filtrat am Rotationsverdampfer bei 100°C vom Lösungsmittel befreit. Man erhält 11,8 g $Fe(H_2O)_y(CF_3SO_3)_2$. 2,5 g $Fe(H_2O)_y(CF_3SO_3)_2$werden in 10 ml Acetonitril gelöst und filtriert. Das Filtrat versetzt man mit 30 ml Diethylether und destilliert dann das Lösungsmittel am Rotationsverdampfer ab. Der Rückstand wird dann bei 100°C im Vakuum getrocknet. Man erhält einen weissen Feststoff, der folgende Analysendaten aufweist:

| | |
|---|---|
| C-Gehalt = | 14,57% |
| N-Gehalt = | 4,97% |
| H-Gehalt = | 2,06%. |

Beispiel 1

In einen 750ml-Sulfierkolben, versehen mit Thermometer, Rückflusskühler, $N_2$-Überleitung und Dosimat, gibt man 90,12 g (1,0 Mol) Butan-1,4-diol und 1,66 g Katalysator A und erwärmt das Reaktionsgemisch auf 100°C Innentemperatur. Unter gutem Rühren werden innerhalb 1 h 160,7 ml (2,05 Mol) Epichlorhydrin zudosiert, und man lässt 1 h nachreagieren. Danach kühlt man das Reaktionsgemisch auf 50°C ab, fügt 200 ml Toluol zu und versetzt die Lösung innerhalb von 5 Minuten (min) mit 16 g (0,2 Mol) 50%-iger wässriger NaOH-Lösung. Danach werden innerhalb von 5 min 76 g (1,9 Mol) pulverisierte NaOH portionenweise zugegeben und die Suspension 30 min gerührt. Man filtriert das Reaktionsgemisch, wäscht es mit 100 ml Toluol und trennt die Phasen. Die organische Phase wird über $MgSO_4$ getrocknet, filtriert und das Filtrat am Rotationsverdampfer vom Lösungsmittel befreit (Badtemperatur 70°C). Man erhält 204,8 g (101% der Theorie) farblosen Butan-1,4-dioldiglycidylether mit folgender Produktqualität: Epoxidgruppengehalt: 8,59 Val/kg (87% d. Th.); totaler Chlorgehalt: 3,8%; hydrolisierbarer Chlorgehalt: 29 ppm (parts per million).

Beispiel 2

In einen 750ml-Sulfierkolben, versehen mit Thermometer, Rückflusskühler, $N_2$-Überleitung und Dosimat, gibt man 90,12 g (1,0 Mol) Butan-1,4-diol zu Katalysator B und erwärmt das Reaktionsgemisch auf 100°C Innentemperatur. Unter gutem Rühren werden innerhalb 1 h 160,7 ml (2,05 Mol) Epichlorhydrin zudosiert, und man lässt 1 h nachreagieren. Danach kühlt man das Reaktiongemisch auf 50°C ab und versetzt die Lösung innerhalb von 5 min mit 16 g (0,2 Mol) 50%-iger wässriger NaOH-Lösung. Danach werden innerhalb von 45 min 76 g (1,9 Mol) pulverisierte NaOH portionenweise zugegeben und die Suspension 30 min gerührt. Man filtriert das Reaktionsgemisch, wäscht es mit 100 ml Toluol und trennt die Phasen. Die organische Phase wird über $MgSO_4$ getrocknet, filtriert und das Filtrat am Rotationsverdampfer vom Lösungsmittel befreit (Badtemperatur 70°C). Man erhält 199,0 g (98% d. Th.) farblosen Butan-1,4-dioldiglycidylether mit folgender Produktqualität: Epoxidgruppengehalt: 8,64 Val/kg (87% d. Th.); totaler Chlorgehalt: 3,4%; hydrolisierbarer Chlorgehalt: 620 ppm.

Beispiel 3

Die Lösung des Katalysators C wird in der Herstellungsapparatur, in der der Destillationsaufsatz durch einen Rückflusskühler ersetzt wird, weiterverwendet. Zur Katalysatorlösung werden 90,12 g (1,0 Mol) Butan-1,4-diol zugegeben, und unter $N_2$ und gutem Rühren werden innerhalb 1 h 160,7 ml (2,05 Mol) Epichlorhydrin zudosiert. Dann lässt man das Reaktionsgemisch 3 h bei Rückfluss nachreagieren. Danach kühlt man auf 50°C ab und versetzt die Lösung innerhalb von 5 min mit 16 g (0,2 Mol) 50%-iger wässriger NaOH-Lösung. Danach werden innerhalb von 45 min 76 g (1,9 Mol) pulverisierte NaOH portionenweise zugegeben und die Suspension 30 min gerührt. Man filtriert das Reaktionsgemisch, wäscht mit 100 ml Toluol und trennt die Phasen. Die organische Phase wird über $MgSO_4$ getrocknet, filtriert und das Filtrat am Rotationsverdampfer bei einer Badtemperatur von 70°C vom Lösungsmittel befreit. Man erhält 203,0 g (100% d. Th.) farblosen Butan-1,4-dioldiglycidylether der folgenden Produktqualität: Epoxidgruppengehalt: 8,78 Val/kg (89% d. Th.); totaler Chlorgehalt: 3,6%; hydrolisierbarer Chlorgehalt: 50 ppm.

Beispiel 4

Die Lösung des Katalysators D wird in der Herstellungsapparatur weiterverwendet. Zur Katalysatorlösung werden 90,12 g (1,0 Mol) Butan-1,4-diol zugegeben, und unter $N_2$ und gutem Rühren werden innerhalb 1 h 160,7 ml (2,05 Mol) Epichlorhydrin zudosiert. Dann lässt man das Reaktionsgemisch 5 h bei Rückfluss nachreagieren. Danach kühlt man auf 50°C ab und versetzt die Lösung innerhalb von 5 min mit 16 g (0,2 Mol) 50%-iger wässriger NaOH-Lösung. Danach werden innerhalb von 45 min 76 g (1,9 Mol) pulverisierte NaOH portionenweise zugegeben und die Suspension 30 min gerührt. Man filtriert das Reaktionsgemisch, wäscht mit 100 ml Toluol und trennt die Phasen. Die organische Phase wird über $MgSO_4$ getrocknet, filtriert und das Filtrat am Rotationsverdampfer bei einer Badtemperatur von 70°C vom Lösungsmittel befreit. Man erhält 205,0 g (101% d. Th.) farblosen Butan-1,4-dioldiglycidylether der folgenden Produktqualität: Epoxidgruppengehalt: 8,68 Val/kg (88% d. Th.); totaler Chlorgehalt: 3,2%; hydrolisierbarer Chlorgehalt: 110 ppm.

Beispiel 5

In einen 750ml-Sulfierkolben, versehen mit Thermometer, Rückflusskühler, $N_2$-Überleitung und Dosimat, gibt man 90,12 g (1,0 Mol) Butan-1,4-diol und 0,75 g Katalysator E und erwärmt das Reaktionsgemisch auf 100°C Innentemperatur. Unter gutem Rühren werden innerhalb 1 h 164,7 ml (2,10 Mol) Epichlorhydrin zudosiert, und man lässt 5 h nachreagieren. Danach kühlt man das Reaktionsgemisch auf 50°C ab, fügt 200 ml Toluol zu und versetzt die Lösung innerhalb von 5 min mit 16 g (0,2 Mol) 50%-iger wässriger NaOH-Lösung. Danach werden innerhalb von 45 min 76 g (1,9 Mol) pulverisierte NaOH portionenweise zugegeben und die Suspension 30 min gerührt. Man filtriert das Reaktionsgemisch, wäscht es mit 100 ml Toluol und trennt die Phasen. Die organische Phase wird über $MgSO_4$ getrocknet, filtriert und das Filtrat am Rotationsverdampfer vom Lösungsmittel befreit (Badtemperatur 70°C). Man erhält 205,4 g (101% der Theorie) leicht gelben Butan-1,4-dioldiglycidylether mit folgender Produktqualität: Epoxidgruppengehalt: 7,61 Val/kg (77% d. Th.); totaler Chlorgehalt: 6,2%; hydrolisierbarer Chlorgehalt: 230 ppm.

Beispiel 6

In einen 750ml-Sulfierkolben, versehen mit Thermometer, Rückflusskühler, $N_2$-Überleitung und Dosimat,

gibt man die Katalysatorlösung F in Butan1,4-diol und erwärmt das Reaktionsgemisch auf 100°C Innentemperatur. Unter gutem Rühren werden innerhalb 1 h 164,7 ml (2,10 Mol) Epichlorhydrin zudosiert, und man lässt das Reaktiongemisch 7 h nachreagieren. Danach kühlt man es auf 50°C ab, fügt 200 ml Toluol zu und versetzt die Lösung innerhalb von 5 min mit 16 g (0,2 Mol) 50%-iger wässriger NaOH-Lösung. Danach werden innerhalb von 45 min 76 g (1,9 Mol) pulverisierte NaOH portionenweise zugegeben und die Suspension 30 min gerührt. Man filtriert das Reaktionsgemisch, wäscht es mit 100 ml Toluol und trennt die Phasen. Die organische Phase wird über $MgSO_4$ getrocknet, filtriert und das Filtrat am Rotationsverdampfer vom Lösungsmittel befreit (Badtemperatur 70°C). Man erhält 196,0 g (97% der Theorie) leicht gelben Butan-1,4-dioldiglycidylether mit folgender Produktqualität: Epoxidgruppengehalt: 8,22 Val/kg (83% d. Th.); totaler Chlorgehalt: 5,0%; hydrolisierbarer Chlorgehalt: 170 ppm.

Beispiel 7

In einen 750ml-Sulfierkolben, versehen mit Thermometer, Rückflusskühler, $N_2$-Überleitung und Dosimat, gibt man 90,12 g (1,0 Mol) Butan-1,4-diol und 0,4 g Katalysator G und erwärmt das Reaktionsgemisch auf 100°C Innentemperatur. Unter gutem Rühren werden innerhalb 1 h 172,43 ml (2,20 Mol) Epichlorhydrin zudosiert und man lässt das Reaktionsgemisch 1 h nachreagieren. Danach kühlt man es auf 50°C ab, fügt 200 ml Toluol zu und versetzt die Lösung innerhalb von 5 min mit 16 g (0,2 Mol) 50%-iger wässriger NaOH-Lösung. Danach werden innerhalb von 45 min 80 g (2,0 Mol) pulverisierte NaOH portionenweise zugegeben und die Suspension 30 min gerührt. Man filtriert das Reaktionsgemisch, wäscht es mit 100 ml Toluol und trennt die Phasen. Die organische Phase wird über $MgSO_4$ getrocknet, filtriert und das Filtrat am Rotationsverdampfer vom Lösungsmittel befreit (Badtemperatur 70°C). Man erhält 220,2 g (109% der Theorie) leicht gelben Butan-1,4-dioldiglycidylether mit folgender Produktqualität: Epoxidgruppengehalt: 7,64 Val/kg (77% d. Th.); totaler Chlorgehalt: 7,5%; hydrolisierbarer Chlorgehalt: 1500 ppm.

Beispiel 8

In einen 750ml-Sulfierkolben, versehen mit Thermometer, Rückflusskühler, $N_2$-Überleitung und Dosimat, gibt man 114,19 g (1,0 Mol) 4-Methylcyclohexanol und 1,7 g Katalysator A und erwärmt das Reaktionsgemisch auf 100°C Innentemperatur. Unter gutem Rühren werden innerhalb 1 h 98,0 ml (1,25 Mol) Epichlorhydrin zudosiert und man lässt das Reaktiongemisch 4 h nachreagieren. Danach kühlt man es auf 50°C ab, gibt 1 g Tetramethylammoniumchlorid zu und versetzt die Lösung innerhalb von 5 min mit 8 g (0,1 Mol) 50%-iger wässriger NaOH-Lösung. Danach werden innerhalb von 45 min 42 g (1,05 Mol) pulverisierte NaOH portionenweise zugegeben und die Suspension 5 h gerührt. Man gibt 200 ml Essigsäureethylester zu, filtriert das Reaktionsgemisch, wäscht es mit 100 ml Essigsäureethylester und trennt die Phasen. Die organische Phase wird über $MgSO_4$ getrocknet, filtriert und das Filtrat am Rotationsverdampfer vom Lösungsmittel befreit (Badtemperatur 60°C). Man erhält 170,7 g (100% der Theorie) farblosen 4-Methylcyclohexanolglycidylether mit folgender Produktqualität: Epoxidgruppengehalt: 5,00 Val/kg (85% d. Th.); totaler Chlorgehalt: 5,0%; hydrolisierbarer Chlorgehalt: 3000 ppm.

Beispiel 9

In einen 750ml-Sulfierkolben, versehen mit Thermometer, Rückflusskühler, $N_2$-Überleitung und Dosimat, gibt man 114,19 g (1,0 Mol) 4-Methylcyclohexanol zu Katalysator B und erwärmt das Reaktionsgemisch auf 100°C Innentemperatur. Unter gutem Rühren werden innerhalb 1 h 98,0 ml (1,25 Mol) Epichlorhydrin zudosiert und man lässt das Reaktiongemisch 3 h nachreagieren. Danach kühlt man es auf 50°C ab, gibt 1 g Tetramethylammoniumchlorid zu und versetzt die Lösung innerhalb von 5 min mit 8 g (0,1 Mol) 50%-iger wässriger NaOH-Lösung. Danach werden innerhalb von 45 min 42 g (1,05 Mol) pulverisierte NaOH portionenweise zugegeben und die Suspension 7 h gerührt. Man gibt 200 ml Essigsäureethylester zu, filtriert das Reaktionsgemisch, wäscht es mit 100 ml Essigsäureethylester und trennt die Phasen. Die organische Phase wird über $MgSO_4$ getrocknet, filtriert und das Filtrat am Rotationsverdampfer vom Lösungsmittel befreit (Badtemperatur 60°C). Man erhält 168,5 g (99% der Theorie) farblosen 4-Methylcyclohexanolglycidylether mit folgender Produktqualität: Epoxidgruppengehalt: 4,93 Val/kg (84% d. Th.); totaler Chlorgehalt: 4,75%; hydrolisierbarer Chlorgehalt: 5900 ppm.

Beispiel 10

In einen 750ml-Sulfierkolben, versehen mit Thermometer, Rückflusskühler, $N_2$-Überleitung und Dosimat,

gibt man 114,19 g (1,0 Mol) 4-Methylcyclohexanol und 0,81 g Katalysator G und erwärmt das Reaktionsgemisch auf 100°C Innentemperatur. Unter gutem Rühren werden innerhalb 1 h 98,0 ml (1,25 Mol) Epichlorhydrin zudosiert und man lässt das Reaktiongemisch 1 h nachreagieren. Danach kühlt man es auf 50°C ab und versetzt die Lösung innerhalb von 5 min mit 8 g (0,1 Mol) 50%-iger wässriger NaOH-Lösung. Danach werden innerhalb von 45 min 42 g (1,05 Mol) pulverisierte NaOH portionenweise zugegeben und die Suspension 5 h gerührt. Man gibt 200 ml Essigsäureethylester zu, filtriert das Reaktionsgemisch, wäscht es mit 100 ml Essigsäureethylester und trennt die Phasen. Die organische Phase wird über $MgSO_4$ getrocknet, filtriert und das Filtrat am Rotationsverdampfer vom Lösungsmittel befreit (Badtemperatur 60°C). Man erhält 184,2 g (108% der Theorie) farblosen 4-Methylcyclohexanolglycidylether mit folgender Produktqualität: Epoxidgruppengehalt: 4,65 Val/kg (79% d. Th.); totaler Chlorgehalt: 5,95%; hydrolisierbarer Chlorgehalt: 330 ppm.

Beispiel 11

In einen 750ml-Sulfierkolben, versehen mit Thermometer, Rückflusskühler, $N_2$-Überleitung und Dosimat, gibt man 78,14 g (0,5 Mol) 4-tert.-Butylcyclohexanol und 0,83 g Katalysator A und erwärmt das Reaktionsgemisch auf 100°C Innentemperatur, wobei das Edukt schmilzt. Unter gutem Rühren werden innerhalb 30 min 39,2 ml (0,5 Mol) Epichlorhydrin zudosiert und man lässt das Reaktiongemisch 6 h nachreagieren. Danach kühlt man es auf 50°C ab, gibt 2 g Tetramethylammoniumchlorid und 200 ml Toluol zu und versetzt die Lösung innerhalb von 5 min mit 4 g (0,05 Mol) 50%-iger wässriger NaOH-Lösung. Danach werden innerhalb von 45 min 19 g (0,475 Mol) pulverisierte NaOH portionenweise zugegeben und die Suspension 2,5 h gerührt. Man filtriert die Suspension , wäscht den Rückstand mit 100ml Toluol und trennt die Phasen. Die organische Phase wird über $MgSO_4$ getrocknet, filtriert und das Filtrat am Rotationsverdampfer vom Lösungsmittel befreit (Badtemperatur 60°C). Man erhält 103,0 g (97% der Theorie) farblosen 4-tert.-Butylcyclohexanolglycidylether mit folgender Produktqualität: Epoxidgruppengehalt: 4,09 Val/kg (87% d. Th.); totaler Chlorgehalt: 1,8%; hydrolisierbarer Chlorgehalt: 270 ppm.

Beispiel 12

In einen 1-Liter-Dreihalskolben, versehen mit Thermometer, Rührer, Rückflusskühler und Tropftrichter, werden unter $N_2$-Gas 50,0 g (0,347 Mol) Cyclohexan-1,4-dimethanol und 1,0 g Katalysator H vorgelegt und auf 130°C Innentemperatur erwärmt. Unter Rühren werden dann innerhalb 1 h 163,8 g (0,69 Mol) Epichlorhydrin zugetropft, und man lässt das Reaktionsgemisch noch 1 h nachreagieren. Anschliessend kühlt man es auf 40°C ab und versetzt die Lösung mit 6,6 g (0,0825 Mol) 50%-iger wässriger Natronlauge. Danach werden innerhalb von 1 h 23,1 g (0,578 Mol) pulverisierte NaOH portionenweise zugegeben. Man rührt die Suspension weiter, versetzt sie mit 155 ml Wasser und filtriert sie. Die organische Phase wird abgetrennt und anschliessend am Rotationsverdampfer bei 100°C unter Vakuum getrocknet. Man erhält 86,2 g (97 % d. Th.) Cyclohexan-1,4-dimethanoldiglycidylether als schwach gelbe Flüssigkeit mit einem Epoxidgehalt von 6,43 Mol/kg (82% d. Th.) und einem totalen Chlorgehalt von 3,9%.

Beispiel 13

In einen 1-Liter-Dreihalskolben, versehen mit Thermometer, Rührer, Rückflusskühler und Tropftrichter, werden 90,0 g (1,0 Mol) Butan-1,4-diol und 2 g einer 50%-igen Lösung von $Fe(H_2O)_y(CF_3SO_3)_2$ in Acetonitril vorgelegt und auf 130°C erhitzt. Unter Rühren werden dann innerhalb 1 h 185,0 g (2,0 Mol) Epichlorhydrin zugetropft, und man lässt das Reaktionsgemisch noch 4,5 h nachreagieren. Anschliessend wird mit 80,0 g (2,0 Mol) NaOH gemäss Beispiel 12 dehydrohalogeniert und das Reaktionsgemisch aufgearbeitet. Man erhält ein dünnflüssiges Harz mit einem Epoxidgehalt von 8,20 Mol/kg (83% d. Th.) und einem Chlorgehalt von 4,3%.

Beispiel 14

Analog Beispiel 12 werden 108,5 g 4-tert.-Butylcyclohexanol und 1,0 g Katalysator H mit 63,8 g Epichlorhydrin unter $N_2$-Gas zur Reaktion gebracht, mit 26,4 g NaOH dehydrohalogeniert und das Reaktionsgemisch aufgearbeitet. Man erhält ein dünnflüssiges Harz mit einem Epoxidgehalt von 3,57 Mol/kg (76% d.Th.) und einem totalen Chlorgehalt von 3,3%.

Beispiel 15

In einen Autoklaven werden 40,8 g (0,4 Mol) 1-Hexanol und 0,66 g Katalysator A vorgelegt und anschliessend 17,6 g (0,4 Mol) Ethylenoxid aufgepresst. Danach wird die Temperatur auf 100°C erhöht, und man lässt das Reaktionsgemisch 12 h bei dieser Temperatur reagieren. Die gaschromatographische Analyse des Reaktionsgemisches ergibt folgende Produkteverteilung (Flächenprozente):

20,4% Edukt,
49,3% Monoaddukt,
22,9% Diaddukt,
6,0% Triaddukt und
0,8% Tetraaddukt.

## Patentansprüche

1. Verfahren zur Herstellung von Anlagerungsprodukten durch Umsetzung eines Alkohols mit einer Epoxidverbindung in Gegenwart eines Katalysators, dadurch gekennzeichnet, dass man einen Alkohol der Formel I

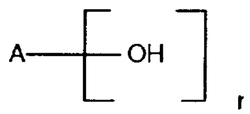
(I),

worin A einen aliphatischen, cycloaliphatischen oder araliphatischen Rest bedeutet und r für eine Zahl von 1 bis 10 steht, mit einer Mono- oder Diepoxidverbindung im Äquivalenzverhältnis von 1:20 bis 20:1, bezogen auf die Hydroxyl- und Epoxidgruppen, in Gegenwart einer Metallkomplexverbindung der Formel II als Katalysator

$$[M^{n+}(L_1)_x(L_2)_y(L_3^{m-})_z][X^{k-}]_{(n-zm)/k} \qquad (II),$$

als Katalysator umsetzt, worin
M ein Metall der Haupt- oder Nebengruppen des Periodensystems,
$L_1$ und $L_2$ schwachgebundene, neutrale, uni- oder multidentale Liganden,
$L_3$ ein starkgebundener, nichtaustauschbarer neutraler oder anionischer, uni- oder multidentaler Ligand,
X ein Anion der folgenden Formeln $BF_4^-$, $PF_6^-$, $AsF_6^-$, $SbF_6^-$, $ClO_4^-$, $JO_4^-$, $NO_3^-$ oder den Sulfonatrest einer perfluorhaltigen Alkansulfonsäure,
n eine Zahl von 1 bis 6,
m null oder eine Zahl von 1 bis 6,
k die Zahl 1 oder 2,
x eine Zahl von 1 bis 1000,
y null oder eine Zahl von 1 bis 1000 und
z null oder eine Zahl von 1 bis 6 bedeuten.

2. Verfahren gemäss Anspruch 1, dadurch gekennzeichnet, dass A in Formel I einen aliphatischen oder cycloaliphatischen Rest darstellt.

3. Verfahren gemäss Anspruch 1, dadurch gekennzeichnet, dass r in Formel I eine Zahl von 1 bis 6 darstellt.

4. Verfahren gemäss Anspruch 1, dadurch gekennzeichnet, dass man als Monoepoxidverbindung ein Epihalogenhydrin oder ein Alkylenoxid einsetzt.

5. Verfahren gemäss Anspruch 1, dadurch gekennzeichnet, dass man als Monoepoxidverbindung ein Epihalogenhydrin einsetzt.

6. Verfahren gemäss Anspruch 5, dadurch gekennzeichnet, dass man Epichlorhydrin, Epibromhydrin oder β-Methylepichlorhydrin als Epihalogenhydrin einsetzt und die erhaltenen Halogenhydrinether durch anschliessende Dehydrohalogenierung mit einer Base zu Glycidylethern der Formel III

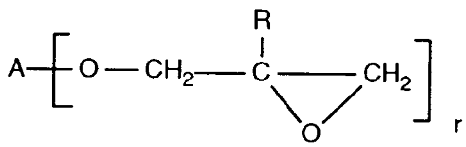

(III),

umwandelt, worin A und r die gleiche Bedeutung wie in Formel I gemäss Anspruch 1 haben, und R für ein Wasserstoffatom oder Methyl steht.

**7.** Verfahren gemäss Anspruch 5, dadurch gekennzeichnet, dass man Epichlorhydrin als Epihalogenhydrin einsetzt.

**8.** Verfahren gemäss Anspruch 5, dadurch gekennzeichnet, dass man auf ein Hydroxyläquivalent des Alkohols der Formel I 0,7 bis 2,0 Mole Epihalogenhydrin einsetzt.

**9.** Verfahren gemäss Anspruch 5, dadurch gekennzeichnet, dass man auf ein Hydroxyläquivalent des Alkohols der Formel I 0,9 bis 1,1 Mole Epihalogenhydrin einsetzt.

**10.** Verfahren gemäss Anspruch 1, dadurch gekennzeichnet, dass man eine Verbindung der Formel II einsetzt, worin
A ein Metall der II., III. oder IV. Hauptgruppe des Periodensystems, ein Metall der Übergangsgruppen, ein Lanthanid oder Actinid,
$L_1$ ein Nitril, Isonitril, Thioether, Phosphin, Ether, Aldehyd, Keton oder eine $sp^3$-stickstoffhaltige Verbindung,
$L_2$ ein Wassermolekül, Nitril, Isonitril, Thioether, Phosphin, Ether, Aldehyd, Keton oder eine $sp^3$-stickstoffhaltige Verbindung,
$L_3$ ein Phosphin, Phosphit, Phosponit, Halogenid, Cyanid, Alkoholat, Thiolat, Carboxylat, Acetylacetonat, ein unsubstituiertes oder durch ein oder mehrere $C_1$-$C_4$-Alkyle oder Tri-($C_1$-$C_4$-alkyl)-silyl substituiertes Cyclopentadienyl oder eine $sp^2$-stickstoffhaltige Verbindung,
X ein Anion der folgenden Formeln $BF_4^-$, $PF_6^-$, $AsF_6^-$, $SbF_6^-$, $ClO_4^-$, $JO_4^-$, $NO_3^-$ oder den Sulfonatrest einer perfluorhaltigen Alkansulfonsäure,
n eine Zahl von 1 bis 3,
m null oder eine Zahl von 1-3,
k die Zahl 1 oder 2,
x eine Zahl von 1 bis 10,
y null oder eine Zahl von 1 bis 10 und
z null oder eine Zahl von 1 bis 3 bedeuten.

**11.** Verfahren gemäss Anspruch 1, dadurch gekennzeichnet, dass man eine Verbindung der Formel II einsetzt, worin
A für Fe, Sn, Zn, Y oder ein Metall der Lanthaniden steht,
$L_1$ ein Nitril, Isonitril, Thioether oder Ether,
$L_2$ ein Wassermolekül, Nitril, Isonitril, Thioether oder Ether,
$L_3$ ein Phosphin, Phosphit, Halogenid oder Carboxylat,
X ein Anion der folgenden Formeln $BF_4^-$, $PF_6^-$, $ClO_4^-$ oder den Trifluormethansulfonatrest bedeuten.

**12.** Verfahren gemäss Anspruch 1, dadurch gekennzeichnet, dass man als Verbindung der Formel II die Metallkomplexverbindung $La(CH_3CN)_x(H_2O)_y(CF_3SO_3)_3$ oder $La(CH_3CN)_x(H_2O)_y(ClO_4)_3$ einsetzt.